(19) Europäisches Patentamt

European Patent Office

Office européen des brevets

(11) **EP 0 617 926 B1**

(12) **EUROPÄISCHE PATENTSCHRIFT**

(45) Veröffentlichungstag und Bekanntmachung des Hinweises auf die Patenterteilung:
**05.08.1998 Patentblatt 1998/32**

(51) Int. Cl.⁶: **A61B 17/92**, B25D 9/14

(21) Anmeldenummer: **94102546.2**

(22) Anmeldetag: **21.02.1994**

(54) **Pneumatisches Schlagwerkzeug**

Pneumatic percussion tool

Outil pneumatique à percusion

(84) Benannte Vertragsstaaten:
**AT BE CH DE DK ES FR GB GR IE IT LI LU MC NL PT SE**

(30) Priorität: **30.03.1993 CH 976/93**

(43) Veröffentlichungstag der Anmeldung:
**05.10.1994 Patentblatt 1994/40**

(73) Patentinhaber:
**IMT INTEGRAL MEDIZINTECHNIK AG**
**6373 Ennetbürgen (CH)**

(72) Erfinder: **Mandanis, Georges**
**CH-6005 Luzern (CH)**

(74) Vertreter:
**Blum, Rudolf Emil Ernst et al**
**c/o E. Blum & Co**
**Patentanwälte**
**Vorderberg 11**
**8044 Zürich (CH)**

(56) Entgegenhaltungen:
EP-A- 0 183 874        DE-C- 3 802 033
FR-A- 2 622 826

**Beschreibung**

Die Erfindung betrifft ein pneumatisches Schlagwerkzeug, insbesondere für den chirurgischen Einsatz, mit einem Gehäuse, einer Zylinder/Kolben-Anordnung mit einem pneumatisch angetriebenen oszillierenden Kolben, und mit einem Werkzeughalter, welcher ausserhalb des Gehäuses eine Werkzeughalterung trägt und innerhalb des Gehäuses zwei Schlagflächen für den Kolben bildet, um wahlweise vorwärtsgerichtete Schläge oder rückwärtsgerichtete Schläge zu erzeugen.

Pneumatische Schlagwerkzeuge, insbesondere für den chirurgischen Einsatz, und dabei z.B. für den Antrieb von Knochenbearbeitungswerkzeugen zur Bearbeitung der Lagerflächen von Knochen zur Aufnahme eines künstlichen Gelenkteiles, sind z.B. aus der Europäischen Patentanmeldung Nr. 452 543 bzw. aus der US-PS 5 152 352 bekannt. Ferner z.B. aus der Internationalen Patentanmeldung WO 89/06516 bzw. aus der US-PS 5 108 400.

Bei den dort gezeigten Schlagwerkzeugen ist zur Umkehrung des Schlagrichtung jeweils der Werkzeughalter gegenüber dem Gehäuse in verschiedene Stellungen bringbar oder im Gehäuse verschieblich geführt. Es hat sich gezeigt, dass die Halterung und die Abdichtung des Werkzeughalters, auf welchen auch erhebliche seitliche Kräfte einwirken, nicht unproblematisch ist. Ferner ist im Falle der EP-A-452 543 die Umkehr der Schlagrichtung nur kompliziert durch Drehung des mit einem Gewinde im Gehäuse gehaltenen Werkzeughalters bewirkbar. Gemäss der WO 89/06516 andererseits erfolgt die Umkehr der Schlagrichtung lediglich durch Ziehen oder Drücken am Gehäuse, was die entsprechende Verschiebung des Werkzeughalters gegenüber dem Gehäuse bewirkt. Es zeigt sich jedoch, dass ein solches Schlagwerkzeug nicht einfach zu bedienen ist, da die Umkehr der Schlagrichtung nicht unabhängig vom Halten des Gehäuses erfolgen kann und auch ungewollt durch zufällige Bewegungen des Gehäuses ausgelöst werden kann.

Es stellt sich daher die Aufgabe, ein pneumatisches Schlagwerkzeug zu schaffen, welches die genannten Nachteile nicht aufweist, und welches insbesondere einfach und definiert in seiner Schlagrichtung umschaltbar ist, ohne dass die Ausübung von Zug oder Druck auf das Gehäuse die Schlagrichtung beeinflusst. Ferner soll das Schlagwerkzeug stabil sein, was insbesondere die Halterung des Werkzeughalters im Gehäuse betrifft, die erheblichen Kräften in verschiedenen Richtungen unterworfen ist.

Diese Aufgabe wird bei einem Schlagwerkzeug der eingangs genannten Art dadurch gelöst, dass der Werkzeughalter im wesentlichen feststehend im Gehäuse gehalten ist, dass der Zylinder als vom Gehäuse getrenntes Bauteil ausgeführt und in diesem verschieblich angeordnet ist, und dass ein manuell betätigbares Umschaltmittel vorgesehen ist, durch dessen Betätigung der Zylinder verschiebbar ist, wobei je nach Verschiebestellung des Zylinders der im Zylinder pneumatisch angetrieben oszillierende Kolben die eine oder die andere der Schlagflächen beaufschlagt.

Die aktive Umschaltung der Schlagrichtung durch Verschiebung des Zylinders erlaubt es einerseits, einen im Gehäuse nicht verschieblichen Werkzeughalter einzusetzen, und andererseits, die Umschaltung unabhängig von Zug- und Druckkräften, welche auf das Gehäuse wirken, auszuführen.

In der EP-A-452 543 ist ein Stufenkolben vorgesehen und die Druckluft wird durch den Kolben gesteuert. Dazu ist insbesondere eine Ueberströmleitung im Gehäuse vorgesehen. Dies hat den Nachteil, dass das Gehäuse in der Herstellung aufwendig ist. Ferner wird dadurch die Baugrösse des Schlagwerkzeuges erhöht, was das Gerät für den Chirurgen unhandlich macht. Vorzugsweise ist daher die Ueberströmleitung gemäss Anspruch 8 als eine Mehrzahl von im Kolben angeordnete Kanäle ausgebildet.

Im folgenden werden Ausführungsbeispiele der Erfindung anhand der Zeichnungen näher erläutert. Dabei zeigen

Figur 1 ein erfindungsgemässes Schlagwerkzeug bei der Erzeugung eines vorwärts gerichteten Schlages;
Figur 2 das Schlagwerkzeug gemäss Figur 1 bei der Erzeugung eines rückwärts gerichteten Schlages, und
Figur 3 einen erfindungsgemässen Kolben für Schlagwerkzeuge in teilweise geschnittener Seitenansicht und in Frontalansicht.

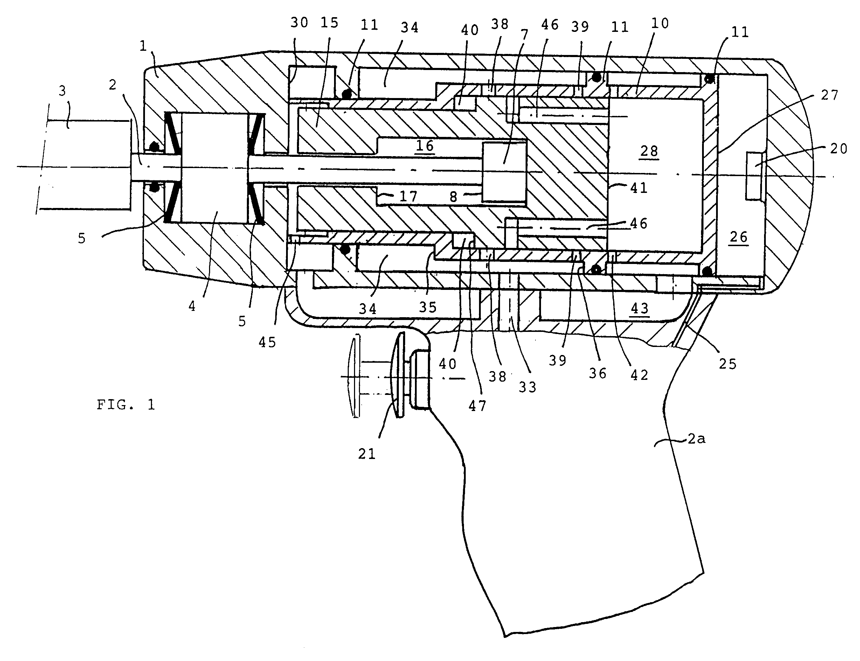

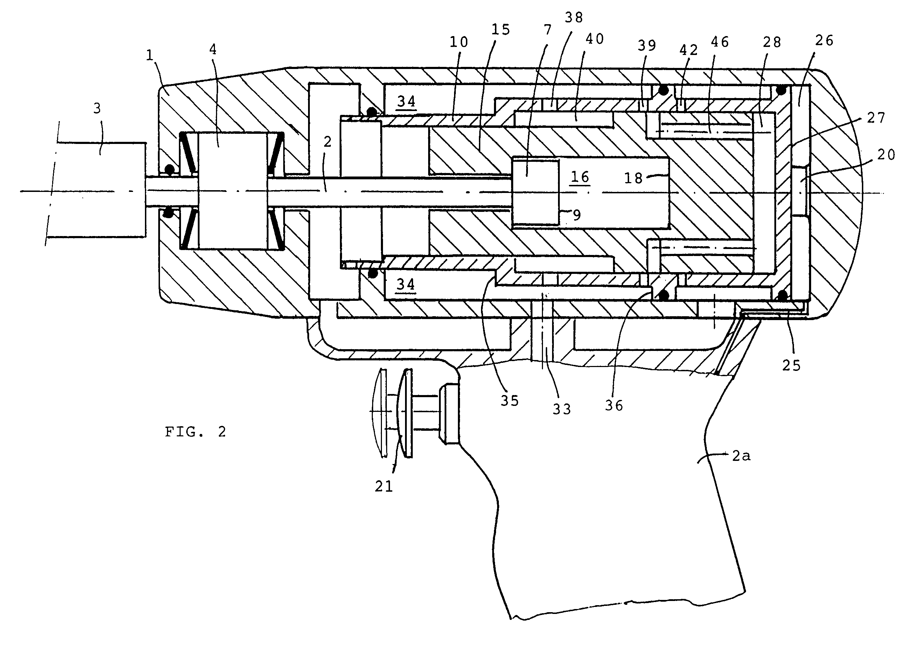

Das in Figur 1 beispielhaft gezeigte Schlagwerkzeug weist ein Gehäuse 1 auf, welches mit einem nur teilweise dargestellten Griff 2a versehen ist. In dem Gehäuse ist ein Werkzeughalter 2 festgehalten. Der Werkzeughalter 2 weist an seinem vorderen Ende eine nur andeutungsweise gezeigte Halterung 3 für ein Knochenbearbeitungswerkzeug, z.B. eine Knochenraspel, auf. Der Werkzeughalter 2 ist im vorderen Teil des Gehäuses 1 durch einen in einer entsprechenden Gehäuseausnehmung angeordneten Lagerblock 4 gegen seitliche Verschiebungen fest gehalten. In axialer Richtung des Werkzeughalters 2 ist dieser bevorzugterweise durch zwischen dem Gehäuse 1 und dem Lagerblock 4 wirkende steife Tellerfedern ebenfalls im wesentlichen fest gehalten. Die Tellerfedern 5 bewirken dabei eine dynamische Isolation des Gehäuses gegen die vom Kolben auf den Werkzeughalter 2 ausgeübten Schläge. Eine solche Isolation des Gehäuses kann auch durch Gummi- oder Kunststoffelemente anstelle der Federn 5 erzielt werden. Auch eine völlig starre Befestigung des Werkzeughalters ist aber möglich. Im Innern des Gehäuses erstreckt sich der Werkzeughalter 2 in einen Hohlraum desselben und weist an seinem Ende einen Kopf 7 auf, welcher mit zwei Schlagflächen 8 und 9 (Figur 2) versehen ist. Im Gehäuseinnern ist ein Zylinder 10 angeordnet. Dieser Zylinder 10 ist im Gehäuseinnern in Längsrichtung verschieblich angeordnet und nimmt in der Regel eine der beiden in den Figuren 1 und 2 dargestellten Endlagen ein. Der Zylinder 10 wird dabei durch mehrere an der Gehäuseinnenwand bzw. an der Zylinderaussenwand befindliche Führungen 11 geführt, an welchen auch Dichtringe angeordnet sein können. Innerhalb des Zylinders ist ein als Hohlkolben ausgestalteter Stufenkolben 15 angeordnet. Der Hohlraum 16 des Kolbens umgreift dabei den Kopf 7 des Werkzeughalters und bildet zwei Schlagflächen 17 und 18 (Figur 2). An der hinteren Innenfläche des Gehäuses ist ein Anschlag 20 vorgesehen. Aussen am Gehäuse ist ein Betätigungselement 21 zur Steuerung des Schlagwerkzeuges vorgesehen. Ferner ist ein nicht gezeigter Anschluss für die Zufuhr von Druckluft vorhanden.

Die Funktion des Schlagwerkzeuges wird zunächst anhand der Figur 1 erläutert, welche den Zylinder 10 in einer solchen Verschiebeposition zeigt, bei welcher das Schlagwerkzeug Schläge in Vorwärtsrichtung auf das Knochenbearbeitungswerkzeug ausübt. Dazu wird bei dem gezeigten Beispiel durch die Bedienungsperson des Schlagwerkzeuges das Betätigungselement 21 in die dargestellte nach rechts geschobene Position gebracht. Dadurch wird mittels eines nicht dargestellten Ventils die Druckluftleitung 25 im Gehäuse 1 über den Druckluftanschluss des Schlagwerkzeuges mit einer Druckluftquelle verbunden. Der Druck dieser Druckluftquelle kann z.B. 7 bar betragen. Ueber die Leitung 25 gelangt nun Druckluft in die Kammer 26, welche im hinteren Teil des Gehäuses 1 durch die Gehäuseinnenwände und durch die Stirnfläche 27 des Zylinders 10 gebildet wird. Die Druckluft in der Kammer 26 übt auf den Zylinder 10 eine Kraft aus, welche den Zylinder in die in Figur 1 dargestellte Verschiebeposition drückt, bei welcher der Zylinder 10 ganz in Richtung auf das Werkzeug hin verschoben ist und mit seinem vorderen Ende an der Gehäuseinnenfläche 30 anliegt. Druckluft wird ferner über die Leitung 33 im Gehäuse 1 in eine zwischen Gehäuse und Zylinder gebildete Kammer 34 geleitet. Diese Druckluft kann ebenfalls mittels des bereits erwähnten Ventils der Leitung 33 zugeführt werden. Es kann dafür aber auch ein separates Ventil vorgesehen sein. Die Druckluft in der Kammer 34 übt einerseits auf den Zylinder über dessen ringförmige Flächen 35 und 36 eine Kraft aus, welche gegen die Kraft gerichtet ist, welche von der Druckluft auf die Fläche 27 des Zylinders ausgeübt wird. Da aber die Zylinderfläche 27 grösser ist als die Ringflächen 35 und 36, ergibt sich trotzdem die in Figur 1 dargestellte Verschiebelage des Zylinders 10. Von der Kammer 34 führen Durchlässe 38 und 39 in das Zylinderinnere. Durch diese Durchlässe 38, 39 wird die zur Erzielung der Kolbenbewegung benötigte Druckluft in das Zylinderinnere geführt. Die Kolbenbewegung erfolgt dabei grundsätzlich in der selben Weise, wie bereits aus der EP-A-452 543 bekannt. In der in Figur 1 gezeigten linken Totpunktlage des Kolbens 15 übt dieser mittels seiner in Figur 2 gezeigten Schlagfläche 18 einen Schlag auf die Schlagfläche 9 des Kopfes 7 des Werkzeughalters 2 aus. Aus der gezeigten linken Totpunktlage heraus wird der Kolben nun zunächst nach rechts beschleunigt, indem die in der Kammer 40, welche von einem Absatz des Zylinders 10 und der Stufe des Stufenkolbens begrenzt wird, befindliche komprimierte Luft expandiert. Bei der Bewegung des Kolbens 15 nach rechts gibt dieser den Durchlass 38 von der Kammer 34 in das Zylinderinnere frei. Druckluft tritt damit in die sich vergrössernde Kammer 40 ein. Die von der Stirnfläche 41 des Kolbens und der Innenwandung des Zylinders begrenzte Kammer 28 ist dabei bei der in der Figur 1 dargestellten Lage des Kolbens zunächst noch drucklos, d.h. auf einem Druckniveau, das dem Umgebungsdruck entspricht. Die Kammer 28 ist nämlich bei der gezeigten Kolbenstellung über die Auslässe 42 in der Zylinderwandung und die Kammer 43 im Gehäuse, welche über nicht dargestellte Gehäuseöffnungen mit der Umgebungsluft in Verbindung steht, noch offen. Bei der Kolbenbewegung nach rechts schliesst aber der Kolben 15 die Auslässe 42 und die in der sich verkleinernden Kammer 28 befindliche Luft wird komprimiert. Durch die Einleitung von Druckluft in die Kammer 40 über den Durchlass 38 ergibt sich aber weiterhin eine Beschleunigung des Kolbens nach rechts. Sobald der Kolben 15 bei seinem Weg zum rechten Totpunkt eine solche Position erreicht hat, dass die Durchlässe 39 von der Kammer 34 in das Zylinderinnere in Verbindung mit dem radial verlaufenden Teil der Ueberströmkanäle 46 im Kolben 15 gelangen, wird Druckluft über die Durchlässe 39 und die Ueberströmkanäle 46 auch in die Kammer 28 geleitet. Es ergibt sich durch die Drucklufteinbringung in die Kammer 28 ein sanftes Abbremsen des Kolbens 15, der damit seinen rechten Totpunkt erreicht und nachfolgend eine Beschleunigung des Kolbens nach links in Richtung auf den Werkzeughalter. Die Expansion in der Kammer 28 beschleunigt den Kolben so lange, bis dieser wieder die Auslässe 42 frei gibt. Bei seiner Bewegung nach links trifft der Kolben wieder mit seiner Schlagfläche 18 auf die Aufprallfläche 9 des Kopfes 7 und es ergibt sich erneut die in der Figur 1 gezeigte Stellung, von welcher aus der Kolben erneut und wie bereits geschildert nach rechts bewegt wird und an seinem rechten Totpunkt sanft abgebremst wird. Bei der in Figur 1 gezeigten Stellung des Zylinders 10 ergibt sich somit ein Vorwärtsschlag des pneumatischen Schlagwerkzeugs. Im vorderen Teil der Zylinderwandung sind ferner noch Durchlässe 45 angeordet, wodurch der vorne im Gehäuse gebildete Raum über Auslässe im Gehäuse 1 mit der Umgebung in Verbindung steht, damit der Kolben nicht durch Kompression in diesem Raum zusätzlich gebremst wird.

Figur 2 zeigt dasselbe Schlagwerkzeug in einer Stellung, bei welcher nur Rückwärtsschläge auf das Knochenbearbeitungswerkzeug ausgeübt werden. Die gleichen Bezugsziffern wie in Figur 1 bezeichnen dabei dieselben Teile. In Figur 2 ist das Betätigungselement 21 in eine mittlere Position gebracht. In diesem Fall ist der Kanal 25 im Gehäuse 1 nicht mit der Druckluftquelle verbunden, sondern drucklos. Damit ist auch die Kammer 26 drucklos. Ueber den Kanal 33 im Gehäuse 1 wird aber weiterhin der Kammer 34 Druckluft zugeführt. Durch die Krafteinwirkung der Druckluft auf die Aussenringflächen 35 und 36 des Zylinders 10 wird dieser in die in Figur 2 gezeigte rechte Position verschoben, wobei die Stirnfläche 27 des Zylinders 10 am Anschlag 20 anliegt. Durch diese Verschiebung des Zylinders 10 im Gehäuse ergibt sich jeweils ein Aufprall des Kolbens 15 mit seiner Schlagfläche 17 auf die Aufprallfläche 8 des Kopfes 7. Dahingegen trifft der Kolben bei seinem Weg nach links nicht mehr mit seiner Schlagfläche 18 auf die Aufprallfläche 9 des Kopfes 7 auf, sondern wird durch die in der Kammer 40 komprimierte Luft sanft abgebremst. Ansonsten erfolgt die Bewegung des Kolbens auf dieselbe Weise wie dies in Figur 1 geschildert worden ist.

In einer dritten Stellung des Betätigungselements 21, welches durch die ganz linke Position dieses Elementes dargestellt ist, kann weiter auch die Druckluftzufuhr durch den Kanal 33 in die Kammer 34 gesperrt werden. In diesem Fall wird der Kolben 15 nicht mehr angetrieben und das Schlagwerkzeug führt keine Schläge mehr aus.

Natürlich ist die gezeigte pneumatische Verschiebung des Zylinders nur als Beispiel zu verstehen. Auch eine mechanische Verschiebung durch einen von Hand zu betätigenden Hebel oder eine andere Art der Verschiebung ist ohne weiteres möglich.

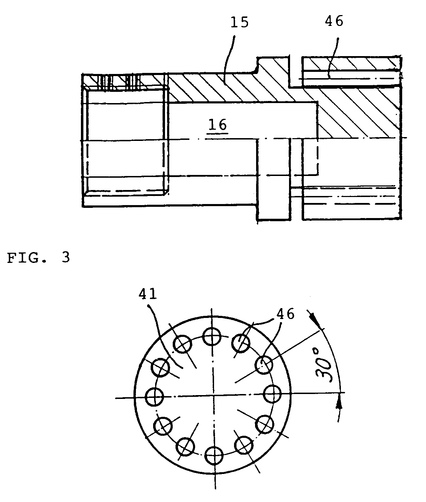

Figur 3 zeigt ferner eine teilweise geschnittene Ansicht des Kolbens. Dieser kann, wie gezeigt, bei einem Schlagwerkzeug gemäss den Figuren 1 und 2 eingesetzt werden. Dieser Kolben, kann aber auch bei einem Schlagwerkzeug, wie es z.B. aus der EP-A-452 543 bekannt ist, eingesetzt werden. Der Kolben weist als Neuheit eine Mehrzahl von Ueberströmkanälen 46 auf. Dadurch kann auf eine Ueberströmleitung im Gehäuse verzichtet werden, was die Konstruktion und Herstellung des Gehäuses vereinfacht. Ferner ergibt sich dadurch ein platzsparender Aufbau des Schlagwerkzeugs. Zudem kann durch eine grössere Zahl von Kanälen die Schlagleistung erhöht werden.

Damit das Verhältnis von vorwärts gerichtetem zu rückwärts gerichtetem Stossimpuls auf das Werkzeug ca. 1 beträgt, erfolgt die Dimensionierung des Schlagwerkzeuges vorzugsweise unter Einhaltung der aus der EP-A-452 543 bekannten folgenden Regeln: Ein erster Parameter A ist definiert wie folgt:

$$A = \frac{A_1}{A_2} \frac{P_0}{P_2}$$

wobei

$A_1 =$ Grösse der oberen Fläche 41 des Kolbens

$A_2 =$ Grösse der ringförmigen Fläche 47 des Kolbens

$P_0 =$ Wert des Umgebungsdrucks (ca. 1 bar)

$P_2 =$ Wert des Drucks der Druckluftquelle (ca. 7 bar)

Zwei weitere Parameter sind definiert wie folgt:

$$V_u = \frac{V_{12}}{V_A} \text{ und } V_o = \frac{V_T}{V_A}$$

wobei $V_A$ das Volumen der Ueberströmkanäle 46 (mit dem Volumen der ringförmigen Nut) plus das Volumen des oberen ZylinderraumeS 28 bei der Kolbenstellung ist, bei welcher der Auslass 42 vom Kolben gerade geschlossen wird;
$V_{12}$ das Volumen der Ueberströmkanäle 46 (mit dem Volumen der ringförmigen Nut) plus des oberen Zylinderraums 28 bei der Kolbenstellung ist, bei welcher die Ueberströmkanäle 46 gerade geöffnet werden und
$V_T$ das Volumen der Ueberströmkanäle 46 (mit Nut) plus des oberen Zylinderraums 28 im oberen Kolbentotpunkt ist.

Das gewünschte Stossimpulsverhältnis von 1 wird erreicht, wenn die Parameter im wesentlichen in folgenden Bereichen liegen:

$$0{,}1 \leq A \leq 0{,}5$$

$$0 \leq V_o \leq V_u \leq 1$$

Vorzugsweise liegen indes die Parameter $V_o$, $V_u$ im Bereich von 0,1 bis 0,8.

**Patentansprüche**

1. Pneumatisches Schlagwerkzeug, insbesondere für den chirurgischen Einsatz, mit einem Gehäuse (1), einer Zylinder/Kolben-Anordnung mit einem pneumatisch angetriebenen oszillierenden Kolben (15), und mit einem Werkzeughalter (2), welcher ausserhalb des Gehäuses eine Werkzeughalterung (3) trägt und innerhalb des Gehäuses zwei Schlagflächen (8,9) für den Kolben bildet, um wahlweise vorwärtsgerichtete Schläge oder rückwärtsgerichtete Schläge zu erzeugen, dadurch gekennzeichnet, dass der Werkzeughalter (2) im wesentlichen feststehend im Gehäuse (1) gehalten ist, dass der Zylinder (10) als vom Gehäuse getrenntes Bauteil ausgeführt und in diesem verschieblich angeordnet ist, und dass ein manuell betätigbares Umschaltmittel (21) vorgesehen ist, durch dessen Betätigung der Zylinder verschiebbar ist, wobei je nach Verschiebestellung des Zylinders der im Zylinder pneumatisch angetrieben oszillierende Kolben (15) die eine oder die andere der Schlagflächen (8,9) beaufschlagt.

2. Schlagwerkzeug nach Anspruch 1, dadurch gekennzeichnet, dass die Verschiebung des Zylinders mittels pneumatischen Drucks erfolgt.

3. Schlagwerkzeug nach Anspruch 2, dadurch gekennzeichnet, dass als Umschaltmittel ein Ventil vorgesehen ist, welches in einer Schaltstellung Druckluft an mindestens eine Ringfläche (35,36) der Zylinderaussenwandung zuführt und in einer anderen Schaltstellung Druckluft an eine Stirnfläche (27) der Zylinderaussenwandung führt.

4. Schlagwerkzeug nach einem der Ansprüche 1 bis 3, dadurch gekennzeichnet, dass zwischen dem Gehäuse und der Zylinderaussenwandung eine erste, stetig mit Druckluft gespiesene Kammer (34) vorgesehen ist, von welcher aus mindestens zwei in Zylinderlängsrichtung beabstandete Durchlässe (38,39) in das Zylinderinnere führen.

5. Schlagwerkzeug nach einem der Ansprüche 3 und 4, dadurch gekennzeichnet, dass die Ringfläche (35, 36) des Zylinders einen Teil der Kammerwandung bildet.

6. Schlagwerkzeug nach einem der Ansprüche 4 oder 5, dadurch gekennzeichnet, dass der Kolben (15) als Stufenkolben ausgebildet ist, der druckluftgesteuert die oszillierende Bewegung ausführt, indem unter Druck stehende Luft dauernd auf eine Kolbenringfläche (47) und nur zeitweise auf die Kolbenstirnfläche (41) einwirkt, und wobei die Kolbenringfläche via den einen Durchlass (38) aus der Kammer mit Druckluft beaufschlagt wird.

7. Schlagwerkzeug nach Anspruch 6, dadurch gekennzeichnet, dass die Kolbenstirnfläche (41) via eine kolbengesteuerte Ueberströmleitung (46) zeitweise mit Druckluft versorgt wird.

8. Schlagwerkzeug nach Anspruch 7, dadurch gekennzeichnet, dass die Ueberströmleitung als eine Mehrzahl von im Kolben angeordnete Kanäle (46) ausgebildet ist, welche Kanäle via den zweiten Durchlass (39) kolbenbewegungsabhängig zeitweise mit der Kammer (34) in Verbindung stehen.

9. Schlagwerkzeug nach einem der Ansprüche 1 bis 8, dadurch gekennzeichnet, dass der Werkzeughalter (2) mittels eines im Gehäuse (1) gehaltenen Lagerblocks (4) gegen seitliche Kräfte unverschieblich gehalten und durch zwischen Gehäuse und Lagerblock eingesetzte Federn (5) oder elastische Gummi- oder Kunststoffelemente in achsialer Richtung fixiert ist.

## Claims

1. Pneumatic battering tool, particularly for surgical use, with a casing (1), a piston/cylinder assembly with a pneumatically driven oscillating piston (15), and with a tool holder (2) which carries tool holding means (3) outside of the casing and which within the casing forms two impact surfaces (8, 9) for the piston in order to selectively generate forward directed or backward directed strikes, characterized in that the tool holder (2) is essentially immobilised in the casing (1), that the cylinder (10) is an element separate from the casing and displaceable within it, and that manually operable switch means (21) are provided which when actuated are able to displace the cylinder, whereby the piston (15) which is urged pneumatically to oscillate in the cylinder will, according to the displaced position of the cylinder, hit either one of the impact surfaces (8, 9) depending on the displacement of the cylinder.

2. Pneumatic battering tool according to claim 1, characterized in that the displacement of the cylinder is obtained through pneumatic pressure.

3. Pneumatic battering tool according to claim 2, characterized in that the switch means comprise a valve which in one position delivers compressed air to at least one annular surface (35, 36) of the outer cylinder wall, and which in another position delivers compressed air to a frontal surface (27) of the outer cylinder wall.

4. Pneumatic battering tool according to one of claims 1 to 3, characterized in that a first chamber (34) continuously fed with compressed air is provided between the casing and the outer wall of the cylinder, and that at least two passages (38, 39) spaced apart in the longitudinal direction of the cylinder lead from the chamber to the inside of the cylinder.

5. Pneumatic battering tool according to one of claims 3 and 4, characterized in that the annular surface (35, 36) of the cylinder is part of the wall of said chamber.

6. Pneumatic battering tool according to one of claims 4 or 5, characterized in that the piston (15) is a stepped piston which oscillates in a way controlled by compressed air which acts continuously on an annular piston surface (47) and only intermittently on the frontal surface (41) of the piston, and in that the annular piston surface is acted upon by compressed air delivered from the chamber through one (38) of the passages.

7. Pneumatic battering tool according to claim 6, characterized in that the frontal surface (41) of the piston is acted upon intermittently by compressed air delivered through a control line (46) controlled by the piston.

8. Pneumatic battering tool according to claim 7, characterized in that the control line comprises several channels (46) in the piston, which channels intermittently communicate with the chamber (34) through the second passage (39), depending on the movement of the piston.

9. Pneumatic battering tool according to one of claims 1 to 8, characterized in that the tool holder (2) is fixedly maintained against the action of transversal forces through a block-shaped bearing means (4) fixed in the casing (1), and that it is maintained in the axial direction by springs (5) or elastic rubber or plastic elements inserted between the casing and the block-shaped bearing means.

**Revendications**

1. Outil de frappe pneumatique, en particulier pour utilisation en chirurgie, avec un boîtier (1), un ensemble cylindre/piston dont le piston (15) oscillant est mû pneumatiquement, et avec un porte-outil (2) supportant un organe porteur d'outil (3) à l'extérieur du boîtier et formant à l'intérieur de celui-ci deux surfaces d'impact (8, 9) pour le piston afin de produire sélectivement des coups dirigés vers l'avant ou vers l'arrière, caractérisé en ce que le porte-outil (2) est maintenu essentiellement immobile dans le boîtier (1), que le cylindre (10) forme un élément distinct du boîtier et est déplaçable dans celui-ci, qu'il est prévu un commutateur (21) à commande manuelle dont l'actionnement déplace le cylindre, le piston (15) oscillant dans le cylindre par action pneumatique frappant l'une ou l'autre des surfaces d'impact (8, 9) en fonction de la position en déplacement du cylindre.

2. Outil de frappe pneumatique selon la revendication 1, caractérisé en ce que le déplacement du cylindre est obtenu par pression pneumatique.

3. Outil de frappe selon la revendication 2, caractérisé en ce que le commutateur à commande manuelle comporte une soupape dirigeant en une position du commutateur de l'air comprimé vers au moins une surface annulaire (35, 36) de la paroi externe du cylindre et dirigeant en une autre position du commutateur de l'air comprimé vers une surface frontale (27) de la paroi externe du cylindre.

4. Outil de frappe selon une des revendications 1 à 3, caractérisé en ce qu'il comporte entre le boîtier et la paroi externe du cylindre une première chambre (34) constamment alimentée en air comprimé et à partir de laquelle au moins deux passages (38, 39) espacés en direction longitudinale du cylindre mènent vers l'intérieur du cylindre.

5. Outil de frappe selon une des revendications 3 et 4, caractérisé en ce que la surface annulaire (35, 36) du cylindre forme une partie de la paroi de la chambre.

6. Outil de frappe selon une des revendications 4 ou 5, caractérisé en ce que le piston (15) est un piston à étages dont le mouvement d'oscillation est commandé pneumatiquement, de l'air comprimé agissant de façon continue sur une surface annulaire (47) du piston et uniquement de façon intermittente sur la surface frontale (41) du piston, la surface annulaire du piston recevant de l'air sous pression en provenance de la chambre à travers l'un des passages (38).

7. Outil de frappe selon la revendication 6, caractérisé en ce que la surface frontale (41) du piston reçoit de façon intermittente de l'air comprimé à travers une conduite de dérivation (46) commandée par le piston.

8. Outil de frappe selon la revendication 7, caractérisé en ce que la conduite de dérivation consiste en plusieurs canaux (46) agencés dans le piston, ces canaux communiquant par intermittence, et en fonction du mouvement du piston, avec la chambre (34), à travers le second passage (39).

9. Outil de frappe selon une des revendications 1 à 8, caractérisé en ce que le porte-outil (2) est maintenu immobile contre des forces transversales au moyen d'un bloc-support (4) maintenu dans le boîtier (1) et est fixé en direction axiale par des ressorts (5) ou des éléments élastiques en caoutchouc ou en matière plastique insérés entre le boîtier et le bloc-support.

FIG. 1

FIG. 2

15   46

16

FIG. 3

41   46

30°